# EUROPÄISCHE PATENTSCHRIFT

(11) **EP 2 499 253 B1**
(45) Veröffentlichungstag und Bekanntmachung des Hinweises auf die Patenterteilung: **22.04.2015**
(21) Anmeldenummer: 10768885.5
(22) Anmeldetag: 23.10.2010
(51) Int. Cl.: C12P 19/12, C12P 19/24, C12N 15/113

(54) **MIKROORGANISMEN MIT GESTEIGERTER SUCROSEMUTASEAKTIVITÄT**
MICROORGANISMS HAVING ENHANCED SUCROSE MUTASE ACTIVITY
MICRO-ORGANISMES PRÉSENTANT UNE ACTIVÉ DE SUCROSE MUTASE ACCRUE

(30) Priorität: 11.11.2009 DE 102009053566
(43) Veröffentlichungstag der Anmeldung: 19.09.2012
(73) Patentinhaber: Südzucker Aktiengesellschaft Mannheim/Ochsenfurt, 68165 Mannheim (DE)
(72) Erfinder: PELZER, Stefan, 64319 Pfungstadt (DE); ZUREK, Christian, 64653 Lorsch (DE); ROSE, Thomas, 67549 Worms (DE); ECK, Jürgen, 64625 Bensheim (DE); WACH, Wolfgang, 67549 Worms (DE); KLINGEBERG, Michael, 67269 Grünstadt (DE); HARMS, Karsten, 67549 Worms (DE)
(74) Vertreter: Wohlfahrt, Jan Günther
(86) Internationale Anmeldenummer: PCT/EP2010/006487
(87) Internationale Veröffentlichungsnummer: WO 2011/057710

(56) Entgegenhaltungen:
- US-A1- 2003 203 468
- CHA J ET AL: "Molecular cloning and functional characterization of a sucrose isomerase (isomaltulose synthase) gene from Enterobacter sp FMB-1", JOURNAL OF APPLIED MICROBIOLOGY, Bd. 107, Nr. 4, Oktober 2009 (2009-10), Seiten 1119-1130, XP002622270, ISSN: 1364-5072
- RICHTER G ET AL: "BIOSYNTHESIS OF RIBOFLAVIN CLONING SEQUENCING AND EXPRESSION OF THE GENE CODING FOR 3 4 DIHYDROXY-2-BUTANONE 4-PHOSPHATE SYNTHASE OF ESCHERICHIA-COLI", JOURNAL OF BACTERIOLOGY, Bd. 174, Nr. 12, 1992, Seiten 4050-4056, XP002622271, ISSN: 0021-9193
- WILLIAM R MCCLEARY: "Application of promoter swapping techniques to control expression of chromosomal genes", APPLIED MICROBIOLOGY AND BIOTECHNOLOGY, SPRINGER, BERLIN, DE, Bd. 84, Nr. 4, 25. Juli 2009 (2009-07-25), Seiten 641-648, XP019737788, ISSN: 1432-0614, DOI: DOI:10.1007/S00253-009-2137-Y
- ALPER HAL ET AL: "Tuning genetic control through promoter engineering", PROCEEDINGS OF THE NATIONAL ACADEMY OF SCIENCES OF THE UNITED STATES (PNAS), NATIONAL ACADEMY OF SCIENCE, US, Bd. 102, Nr. 36, 6. September 2005 (2005-09-06), Seiten 12678-12683, XP002428722, ISSN: 0027-8424, DOI: DOI:10.1073/PNAS.0504604102
- SAWITZKE JAMES A ET AL: "Recombineering: in vivo genetic engineering in E. coli, S. enterica, and beyond", METHODS IN ENZYMOLOGY; [METHODS IN ENZYMOLOGY], ACADEMIC PRESS INC, SAN DIEGO, CA, US, Bd. 421, 1. Januar 2007 (2007-01-01), Seiten 171-199, XP008124026, ISSN: 0076-6879, DOI: DOI:10.1016/S0076-6879(06)21015-2 [gefunden am 2007-03-09]
- DE OLIVA-NETO P ET AL: "Isomaltulose production from sucrose by Protaminobacter rubrum immobilized in calcium alginate", BIORESOURCE TECHNOLOGY, ELSEVIER BV, GB, Bd. 100, Nr. 18, 1. September 2009 (2009-09-01), Seiten 4252-4256, XP026130078, ISSN: 0960-8524, DOI: DOI:10.1016/J.BIORTECH.2009.03.060 [gefunden am 2009-05-01]
- HERRERO M ET AL: "TRANSPOSON VECTORS CONTAINING NON-ANTIBIOTIC RESISTANCE SELECTION MARKERS FOR CLONING AND STABLE CHROMOSOMAL INSERTION OF FOREIGN GENES IN GRAM-NETATIVE BACTERIA", JOURNAL OF BACTERIOLOGY, AMERICAN SOCIETY FOR MICROBIOLOGY, WASHINGTON, DC; US, Bd. 172, Nr. 11, 1. November 1990 (1990-11-01), Seiten 6557-6567, XP000572232, ISSN: 0021-9193 in der Anmeldung erwähnt
- DATABASE Geneseq [Online] 4. März 2010 (2010-03-04), "S. enterica 3,4 dihydroxy-2-butanone-4-phosphate synthase promoter.", XP002622272, gefunden im EBI accession no. GSN:AXU25741 Database accession no. AXU25741 -& WO 2009/152480 A2 (VIVOCURE INC [US]; ARRACH NABIL [US]; MCCLELLAND MICHAEL [US]) 17. Dezember 2009 (2009-12-17)

## Beschreibung

Die Erfindung betrifft die biotechnologische Herstellung von Isomaltulose und Isomaltulose-haltigen Zusammensetzungen und stellt verbesserte Mittel, besonders mikrobielle Zellen, dazu bereit.

Die Disaccharide Isomaltulose (Palatinose) und Trehalulose können durch Umlagerung (Isomerisierung) aus Saccharose (Rohrzucker, Rübenzucker) hergestellt werden. Saccharose-Isomerisierungsprodukte, besonders Isomaltulose, werden zunehmend als Saccharoseaustauschstoffe in Lebensmitteln und verwandten Produkten eingesetzt. Isomaltulose ist ein akariogener Zucker mit niedrigem glykämischen Index und ist seit 2005 als Lebensmittel oder Lebensmittelzutat zur Verwendung in Lebensmitteln im Verkehr zugelassen. Daneben sind Isomaltulose oder Isomaltulose-haltige Zusammensetzungen, die aus Saccharose gewonnen werden können, Ausgangsstoffe für die Herstellung des Zuckeralkohols Isomalt, einem razemischen Gemisch aus 1,6-GPS (6-O-α-D-Glucopyranosyl-D-sorbit) und 1,1-GPM (1-O-α-D-Glucopyranosyl-D-mannit), sowie von Abwandlungen davon, besonders von GPS- oder GPM-angereicherten Gemischen. Isomalt ist inzwischen als Zuckeraustauschstoff in Lebensmitteln und verwandten Produkten etabliert. Die Umwandlung von Isomaltulose zu Isomalt und verwandten Produkten erfolgt bekanntermaßen durch katalytische Hydrierung, alternativ durch biotechnologische Umwandlung.

Die biotechnologische Herstellung von Isomaltulose und Trehalulose geschieht bekanntermaßen durch enzymatische Isomerisierung der Saccharose unter Verwendung von vorzugsweise immobilisierten Bakterienzellen oder Fragmenten davon. Die Isomerisierung erfolgt dabei über die Aktivität des bakteriellen Enzyms Sucrosemutase, E.C. 5.4.99.11 (synonym: Sucroseisomerase, Saccharosemutase, Saccharoseisomerase, Isomaltulosesynthase). Mikroorganismen, die bekanntermaßen Sucrosemutaseaktivität besitzen und in biotechnologischen Prozessen eingesetzt werden können, sind insbesondere *Protaminobacter rubrum, Erwinia rhapontici, Pseudomonas mesoacidophila, Pantoea dispersa* und *Serratia plymuthica.* Deren Sucrosemutase ist unter den Bezeichnungen SmuA, Pal I, SmtA, MutB, SIM und weiteren bekannt. Die bakteriellen Sucrosemutasen werden durch ein chromosomales Gen kodiert. Die Genexpression wird über ein Sucrosemutase-Promotorelement gesteuert. Im Folgenden werden diese Sucrosemutasen einheitlich stellvertretend durch die Bezeichnung "SmuA" der Sucrosemutase aus *P*. *rubrum* benannt, ohne dass diese auf die Sucrosemutase aus *P. rubrum* beschränkt wären. In *P. rubrum* wird SmuA durch das chromosomale Gen mit der Bezeichnung *smuA* kodiert; die Expression wird über ein *smuA-*Promotorelement gesteuert. Im Folgenden steht auch die Bezeichnung *smuA* stellvertretend für die Sucrosemutase-Gene anderer Organismen.

Aus der EP 0751218 A und WO 95/20047 A sind genetisch modifizierte transgene Mikroorganismen bekannt, die Sucrosemutaseaktivität aufweisen und worin durch Mutagenese der Zelle mindestens ein Gen, das eine Enzymaktivität des Saccharosestoffwechsels kodiert, inaktiviert ist.

Die Rate der Umsetzung von dem Substrat Saccharose zu Isomaltulose und gegebenenfalls zusätzlich zu Trehalulose in bekannten Mikroorganismen ist verbesserungswürdig. Daneben ist die Sucrosemutaseaktivität in bekannten Mikroorganismen auch von der Konzentration oder Anwesenheit von Saccharose im Kulturmedium abhängig, was die biotechnologische Verfahrensführung verkompliziert. Es bestehen zudem gesetzliche Beschränkungen und Auflagen für gentechnisch veränderte Organismen (GmO), zum Beispiel die deutsche Gentechnik-Sicherheitsverordnung (GenTSV). Neben der Bereitstellung von Mikroorganismen, mit denen eine verbesserte IsomaltuloseAusbeute zu erzielen ist, besteht auch der Wunsch nach verbesserten Mikroorganismen, die gesetzlichen Auflagen nicht unterliegen.

Das der Erfindung zugrundeliegende technische Problem bestand darin, verbesserte Verfahren und Mittel, besonders verbesserte Mikroorganismen zur biotechnologischen Herstellung von Isomaltulose und Isomaltulose-haltigen Zusammensetzungen bereitzustellen, welche die aus dem Stand der Technik bekannten Nachteile überwinden.

Die Erfindung löst das technische Problem vollständig durch die Bereitstellung einer neuartigen mikrobiellen Zelle, welche gegenüber dem, bevorzugt ansonsten unmodifizierten, Wildtyp dieser Zelle eine mindestens um den Faktor 2, bevorzugt um Faktor 2,5, besonders bevorzugt um Faktor 3 oder 4 oder 5 oder mehr, erhöhte Isomaltulose-Bildungsrate, das heißt Syntheserate, aufweist.

In diesem Zusammenhang stellt die Erfindung bevorzugt eine Zelle bereit, die eine gegenüber ihrem Wildtyp mindestens um den Faktor 2, bevorzugt Faktor 2,5, besonders bevorzugt um Faktor 3 oder 4 oder 5 oder mehr, gesteigerte Aktivität, das heißt besonders Volumenaktivität, der von der Zelle exprimierten, Sucrosemutase aufweist. In bevorzugter Ausführung weist die erfindungsgemäße Zelle gegenüber ihrem Wildtyp eine substratunabhängige, besonders eine von der Saccharosekonzentration unabhängige Expression der Sucrosemutase auf.

Die Erfindung sieht zur Bereitstellung der erfindungsgemäßen Zelle vor, den nicht kodierenden Promotorbereich des Gens *smuA,* das die Sucrosemutase SmuA kodiert, in dem, bevorzugt ansonsten unmodifizierten Wildtyp der Zelle durch einen anderen starken, bevorzugt eigenen endogenen oder homologen Promotor oder ein funktionelles Promotorfragment davon (=Austauschpromotor) zu ersetzen. Die Erfinder fanden überraschend hochwirksame homologe und besonders endogene, das heißt organismeneigene, Promotoren sowie Fragmente davon, womit die Expression des *smuA*-Gens und infolgedessen besonders die Konzentration der Sucrosemutase im Periplasma der Zelle und damit besonders die Volumenaktivität und damit besonders die Isomaltulose-Bildungsrate gegenüber dem Wildtyp der Zelle gesteigert werden kann.

Gegenstand der Erfindung ist also eine Zelle, die vorzugsweise im Genom ein, bevorzugt endogenes, Sucrosemutasegen (*smuA*-Gen) aufweist, das eine Sucrosemutase (SmuA) kodiert, wobei ein *smuA-*Sucrosemutase-Promotorelement oder -Promotor (*smuA*-Promotor), das heißt mindestens eine die Expression dieses Gens steuernde oder regulierende Einheit, durch mindestens einen anderen Promotor oder dessen Fragment (Austauschpromotor) ersetzt ist, wobei der Austauschpromotor ausgewählt ist aus: (a) einem von dem *smuA-*Promotor verschiedenen endogenen Promotor aus dem eigenen Stamm der Zelle (eigener, endogener Promotor), und zwar dem Promotor des Gens der 3,4-Dihydroxy-2-butanon-4-phosphat Synthese (*ribB*), (b) einem zu dem endogenen Promotor nach (a) homologen Promotor aus einem fremden Spenderstamm (fremder, homologer Promotor) und (c) einem funktionellen Promotorfragment von (a) oder (b), wobei der Austauschpromotor durch ein Polynucleotidmolekül charakterisiert ist, das ausgewählt ist aus der Gruppe bestehend aus Polynucleotidmolekülen mit einer Nucleotidsequenz nach SEQ ID NO: 2 bis SEQ ID NO: 3 und homologen Sequenzen davon mit zumindest 85% Sequenzübereinstimmung, das die Expression dieses *smuA*-Gens steuern oder regulieren, insbesondere gegenüber dem Wildtyp der Zelle steigern, kann.

In einer besonders bevorzugten Ausführung ist diese Zelle ein Selbstklonierungsstamm, der durch Selbstklonierung mit zelleigenen (endogenen) Gen-Sequenzen erzeugt wurde. Die erfindungsgemäße Zelle ist also bevorzugt ein Promotor-Reorganisationsstamm. Erfindungsgemäß wird dabei mittels einer Kopie eines nicht proteinkodierenden anderen, bevorzugt stammeigenen (endogenen) regulatorischen Fragments, das heißt Promotor oder dessen funktionelles Fragment, das durch Promotorreorganisation zur Expression der eigenen Saccharosemutase eingesetzt wird, ein Stamm mit den verbesserten Eigenschaften erzeugt. Vorteilhafterweise bildet dieser Promotorreorganisationsstamm kein neues Protein; er vermittelt bevorzugt ausschließlich die Expression größerer Mengen der zelleigenen Saccharosemutase SmuA, die ja in identischer Form auch vom Wildtyp der Zelle exprimiert wird. Bevorzugt sind also keine der Zelle fremden Gene und/oder Genfragmente, und vorzugsweise keine fremden kodierenden und/oder nicht kodierenden Polynucleinsäuremoleküle in der erfindungsgemäßen Zelle enthalten, und zwar weder episomal noch chromosomal. Es versteht sich, das zum Herstellen des Selbstklonierungsstamms in dieser erfindungsgemäßen Ausführungsvariante temporär, intermediär Vektorelemente in die Zelle eingebracht werden, die sich dort aber nicht dauerhaft manifestieren, sondern erfindungsgemäß bevorzugt durch Selektion auf das zweite Rekombinationsereignis aus der Zelle wieder entfernt werden. Die Gensequenzen werden bevorzugt narbenfrei ausgetauscht. Die erfindungsgemäße Lehre verzichtet in dieser Ausführung auf das dauerhafte Einschleusen von genetischem Material aus einem fremden "Spenderorganismus" laut GenTSV. Dadurch wird eine verbesserte mikrobielle Zelle bereitgestellt, die laut geltenden gesetzlichen Vorschriften und Richtlinien nicht als "gentechnisch veränderter Organismus" (GVO) gilt. Die für GVOs geltenden Sicherheitsvorschriften, Verwendungsbeschränkungen und Auflagen brauchen nicht oder nicht vollumfänglich beachtet zu werden, was vor allem die wirtschaftliche Verwertung der erfindungsgemäßen Zelle erleichtert.

In einer alternativen zweiten Ausführung stellt die Erfindung eine rekombinante Zelle bereit, die einen homologen Promotor aufweist. Diese Zelle trägt an Stelle des nativen *smuA*-Promotors zumindest einen anderen Promotor, der aus einem homologen Spenderorganismus stammt, oder funktionelles Promotorfragment davon. Dieser ist bevorzugt homolog zu dem endogenen Austauschpromotor der ersten Ausführung der Erfindung.

Gemäß Erfindung ist der Austauschpromotor ausgewählt aus oder charakterisiert durch:
- Promotor des Gens der 3,4-Dihydroxy-2-butanon-4-phosphat Synthase *ribB.*

Damit im Zusammenhang können die folgenden Promotoren stehen:
- Promotor des das "outer membrane protein" kodierenden Gens *ompA,*
- Promotor des den "putativen Transkriptionsaktivator ECA2934" kodierenden Gens,
- Promotor des Gens der Ribonuclease E *rne,*
- Promotor des Operons des 50S ribosomalen L21-Proteins,
- Promotor des Operons des Kälteschockproteins CspE,
- Promotor des Operons des 50S ribosomalen L28-Proteins, und
- Promotor des Gens der NAD abhängigen Epimerase-Dehydratase.

In einer bevorzugten Variante ist als Austauschpromotor ein funktionelles Promotorfragment des gesamten Promotors vorgesehen, das in der Lage ist, die Expression des *smuA*-Gens erfindungsgemäß zu steuern.

Die Tabellen 1 A und 1 B listen Austauschpromotoren und deren charakterisierende Quellen auf.

**Tabelle 1 A:**

| *Größe (bp)* | *Promotorfragment charakterisiert in Quelle; Homologie zu:* | *SEQ ID NO:* |
|---|---|---|
| 321 | *P. rubrum* ribB Promotorbereich (3,4-Dihydroxy-2 butanon 4-phosphatsynthase) | 2 |
| 313 | Promotorbereich ribB (3,4-Dihydroxy-2 butanon 4-phosphatsynthase, *Spro_4286*) aus *Serratia proteamaculans* 568 Identities = 298/313 (95%) | 3 |
| 228 | *P. rubrum* ompA Promotorbereich | 4 |
| 180 | ompA Promotor aus S. *proteamaculans* (Spro_1754) Identities = 178/180 (98%) | 5 |
| 178 | ompA Promotor aus S. *marcescens* (emb X00618.1) Identities = 173/180 (96%) | 6 |
| 109 | *P. rubrum* intergenischer Bereich zwischen "hypothetical protein ECA2934" und "putative transcriptional regulator" | 7 |
| 59 | Promotorbereich vor "ECA2934 putative transcriptional regulator [*Pectobacterium atrosepticum* SCR11043]" aus *Erwinia carotovora subsp. atroseptica* SCR11043 (emb BX950851.1) Identities = 51/59 (86%) | 8 |
| 209 | *P. rubrum* intergenischer Bereich zwischen "ribonuclease E"" und "23S rRNA pseudouridylate synthase C"; Promotoraktivität in Richtung Ribonuklease | 9 |
| 210 | intergenische Region zwischen "ribonuclease E" (Spro_1898) und 23S rRNA pseudouridylate synthase C (Spro_1899) in S. *proteamaculans* (gb CP000826.1) Identities = 203/210 (96%) | 10 |
| 165 | Promotorbereich des "ribonuclease E (rne)" Gens aus *Serratia marcescens* (gb AF259269.1, AF259269) Identities = 158/165 (95%) | 11 |

**Tabelle 1B:**

| *Größe (bp)* | *Promotorfragment charakterisiert in Quelle; Homologie zu:* | *SEQ ID NO:* |
|---|---|---|
| 242 | *P. rubrum* intergenischer Bereich: flankierende Gene "Octaprenyldiphosphat Synthase" und "50S ribosomal protein L21" | 12 |
| 242 | Promotorregion des "50S ribosomal protein L21" (Spro_0473) aus *S. proteamaculans* (gb CP000826.1) Identities = 236/242 (97%) | 13 |
| 120 | *P. rubrum* Promotorbereich des "cold shock protein CspE" | 14 |
| 118 | Promotorregion des "cold shock protein CspE" (Spro_1189) aus *S*. *proteamaculans* (gb CP000826.1) Identities = 111/118 (94%) | 15 |
| 115 | Promotorregion des "cold shock protein CspE" Identities = 94/115 (81%) | 16 |
| 238 | *P. rubrum* Promotorbereich "50S ribosomales Protein L28" | 17 |
| 238 | Promotorregion des "50S ribosomal protein L28" (Spro_4481; gene "rpmB") aus *S*. *proteamaculans* (gb CP000826.1) Identities = 231/238 (97%) | 18 |
| 238 | Bereich des "waa gene clusters" aus *S. marcescens* Stamm N28b (gb U52844.3 SMU52844) Identities = 226/238 (94%) | 19 |
| 152 | *P. rubrum* Promotorbereich "NAD abhängige Epimerase-Dehydratase" | 20 |
| 86 | Promotorregion der "NAD abhängigen Epimerase-Dehydratase (Spro_2372)" aus *S. proteamaculans* (gb CP000826.1) Identities = 73/86 (84%) | 21 |

Ein erfindungsgemäß eingesetztes Austauschfragment, das von dem Promotor des Gens der 3,4-Dihydroxy-2-butanon-4-phosphat Synthase *ribB* oder einer homologen Region stammt, ist charakterisiert durch die Nucleotidsequenz, ausgewählt aus: SEQ ID NO: 2 und SEQ ID NO: 3. In einer bevorzugten Ausführung ist die Zelle eine *P. rubrum* Zelle, die mit dem Austauschfragment nach SEQ ID NO: 2 aus *P. rubrum* eigenkloniert ist.

In Zusammenhang mit der Erfindung steht und ein Austauschfragment, das von dem Promotor für das "outer membrane protein" kodierende Gen *ompA* oder einer homologen Region stammt, ist charakterisiert durch die Nucleotidsequenz, ausgewählt aus: SEQ ID NO: 4, SEQ ID NO: 5 und SEQ ID NO: 6 und eine *P. rubrum* Zelle, die mit dem Austauschfragment nach SEQ ID NO: 4 aus *P. rubrum* eigenkloniert ist.

In Zusammenhang mit der Erfindung steht und ein Austauschfragment, das von dem Promotor des Gens des putativen Transkriptionsaktivators ECA2934 oder einer homologen Region stammt, ist charakterisiert durch die Nucleotidsequenz, ausgewählt aus: SEQ ID NO: 7 und SEQ ID NO: 8 und eine *P. rubrum* Zelle, die mit dem Austauschfragment nach SEQ ID NO: 7 aus *P. rubrum* eigenkloniert ist.

In Zusammenhang mit der Erfindung steht und ein Austauschfragment, das von dem Promotor des Gens der Ribonuclease E *rne* oder einer homologen Region stammt, ist charakterisiert durch die Nucleotidsequenz, ausgewählt aus: SEQ ID NO: 9, SEQ ID NO: 10 und SEQ ID NO: 11 und eine *P*. *rubrum* Zelle, die mit dem Austauschfragment nach SEQ ID NO: 9 aus *P. rubrum* eigenkloniert ist.

In Zusammenhang mit der Erfindung steht und ein Austauschfragment, das von Promotor des Operons des 50S ribosomalen L21-Proteins oder einer homologen Region stammt, ist charakterisiert durch die Nucleotidsequenz, ausgewählt aus: SEQ ID NO: 12 und SEQ ID NO: 13 und eine *P. rubrum* Zelle, die mit dem Austauschfragment nach SEQ ID NO: 12 aus *P. rubrum* eigenkloniert ist.

In Zusammenhang mit der Erfindung steht und ein Austauschfragment, das von dem Promotor des Operons des Kälteschockproteins CspE oder einer homologen Region stammt, ist charakterisiert durch die Nucleotidsequenz, ausgewählt aus: SEQ ID NO: 14, SEQ ID NO: 15 und SEQ ID NO: 16 und eine *P. rubrum* Zelle, die mit dem Austauschfragment nach SEQ ID NO: 14 aus *P. rubrum* eigenkloniert ist.

In Zusammenhang mit der Erfindung steht und ein Austauschfragment, das von dem Promotor des Operons des 50S ribosomalen L28-Proteins oder einer homologen Region stammt, ist charakterisiert durch die Nucleotidsequenz, ausgewählt aus: SEQ ID NO: 17, SEQ ID NO: 18 und SEQ ID NO: 19 und eine *P. rubrum* Zelle, die mit dem Austauschfragment nach SEQ ID NO: 17 aus *P. rubrum* eigenkloniert ist.

In Zusammenhang mit der Erfindung steht und ein Austauschfragment, das vom Promotor des Gens der NAD abhängigen Epimerase-Dehydratase oder einer homologen Region stammt, charakterisiert durch die Nucleotidsequenz, ausgewählt aus: SEQ ID NO: 20 und SEQ ID NO: 21 und eine *P*. *rubrum* Zelle, die mit dem Austauschfragment nach SEQ ID NO: 20 aus *P. rubrum* eigenkloniert ist.

Der erfindungsgemäße endogene oder alternativ homologe Austauschpromotor ist also durch ein Polynucleotid charakterisiert oder bevorzugt daraus gebildet, das eine Nucleotidsequenz vorzugsweise aufweist oder daraus besteht, die ausgewählt ist aus der Gruppe der erfindungsgemäßen endogenen beziehungsweise homologen Austauschsequenzen, bestehend aus SEQ ID NO: 2 und SEQ ID NO: 3 und deren funktionellen Abwandlungen und Fragmenten. Bevorzugt ist als Austauschpromotor gerade dieses Polynucleotid eingesetzt; alternativ bevorzugt enthält er dieses Polynucleotid als das wesentliche oder charakterisierende funktionelle Element. Die Erfindung betrifft somit auch dem Fachmann daraus ohne weiteres zugängliche funktionelle Fragmente oder Abwandlungen dieser konkret genannten Polynucleotide, wenn und soweit diese als Promotor oder allgemein als die Expression regulierendes Element für das Sucrosemutase-Gen *smuA* fungieren können.

Die Erfindung betrifft dabei funktionelle Polynucleotidmoleküle mit dazu homologen Sequenzen, die zumindest 85%, bevorzugt mehr als 90 %, oder mehr als 95 %, oder mehr als 97 % Sequenzübereinstimmung (Identities) mit den vorstehend konkret genannten Sequenzen SEQ ID NO: 2 oder SEQ ID NO: 3 aufweisen. Der Fachmann kennt die experimentellen Verfahren und Algorithmen zur Bestimmung der Sequenzhomologie und der funktionellen Analogie.

Unter "funktionellem Fragment" wird insbesondere ein Polynucleotidmolekül mit einer gegenüber der konkret angegebenen Sequenz verkürzten Sequenz verstanden, die zumindest das eigentliche Promotormotiv, das heißt, die die Expression des *smuA*-Gens vermittelnde regulatorische Einheit enthält oder bevorzugt daraus besteht. Der Fachmann kennt geeignete routinemäßige Screeningverfahren, um geeignete funktionelle Fragmente im Sinne der Erfindung zu ermitteln. Diese ohne Weiteres ermittelbaren funktionellen Fragmente sind ebenfalls Gegenstand dieser Erfindung. Bevorzugt sind die Fragmente gegenüber dem hier konkret genannten Nucleotidmolekül um etwa 100 bp, 50 bp, 20 bp, 10 bp, 5 bp oder 2 bp oder um etwa 50, 20, 10, 5 oder 2 der ursprünglichen Basenzahl verkürzt.

Unter "funktioneller Abwandlung" der Polynucleotidmoleküle mit den konkret genannten Sequenzen wird insbesondere ein als regulatorische Einheit, welche die Expression des *smuA*-Gens vermitteln kann, fungierendes Polynucleotidmolekül verstanden, das sich vor allem unmittelbar durch Austausch (Substitution), Weglassen (Deletion) oder Ergänzen (Addition) von einem oder mehreren Nucleotiden von dem konkreten Polynucleotidmolekül unterscheidet. Der Fachmann kennt geeignete Screening-Verfahren, um solche abgewandelten Moleküle, die für die Ausführung der Erfindung geeignet sind, ohne Weiteres zu finden. Bevorzugt sind diese Abwandlungen durch Substitution, Deletion und/oder Addition von 1 oder mehreren, 2, 3, 4, 5, oder etwa 10, 20, 50 oder 100 oder mehr Nucleotiden von den konkret genannten Polynucleotidmolekülen abgeleitet.

Diese funktionellen Abwandlungen oder Fragmente stammen in einer bevorzugten Variante der Erfindung endogen aus der Wirtszelle, besonders bevorzugt aus deren Genom. In einer alternativen Variante sind diese Abwandlungen oder Fragmente homologe Elemente aus anderen Organismen.

Daneben umfasst die Erfindung auch Polynucleotidmoleküle, die sich als regulatorische Einheiten zur Vermittlung der Expression des *smuA*-Gens in der Zelle eignen und als Austauschpromotormolekül erfindungsgemäß eingesetzt werden können, welche sich durch randomisierte Synthese oder Abwandlung der benannten Austauschpromotoren in an sich bekannter Weise ergeben. Diese kann der Fachmann durch routinemäßiges Vorgehen aus der hierin beschriebenen Lehre in an sich bekannter Weise bereitstellen.

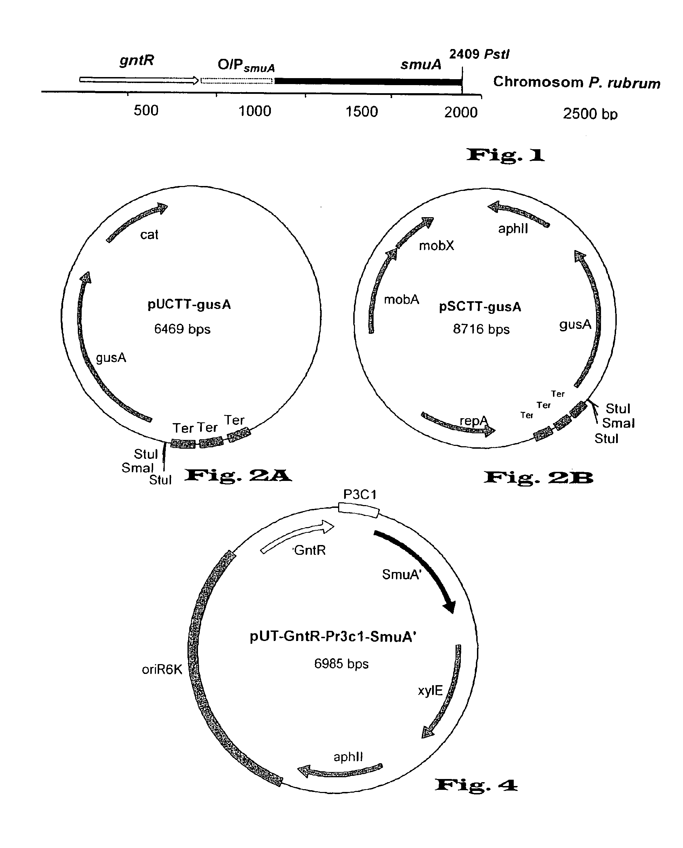

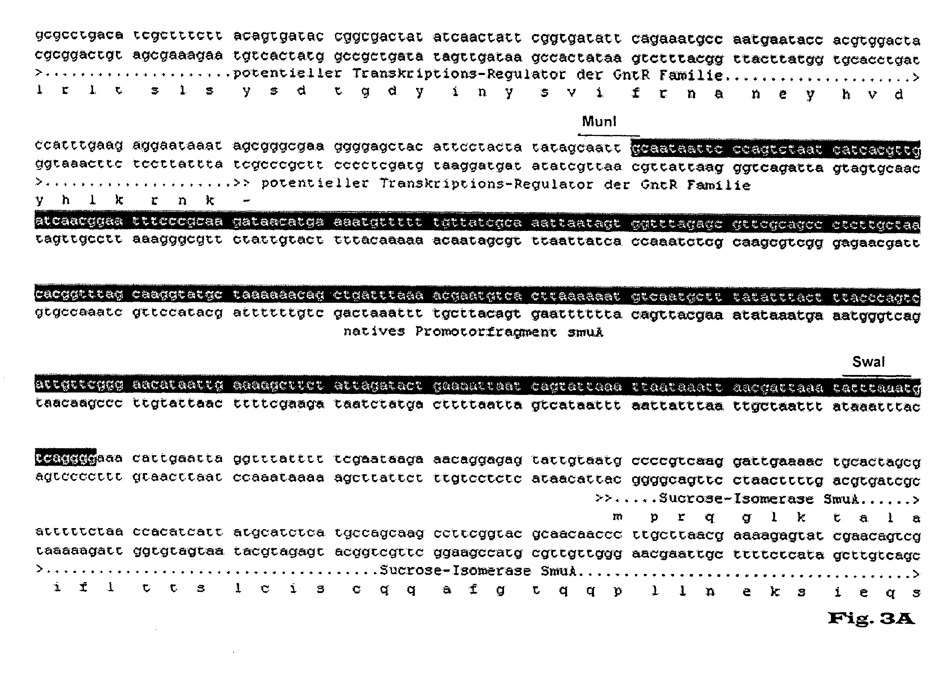

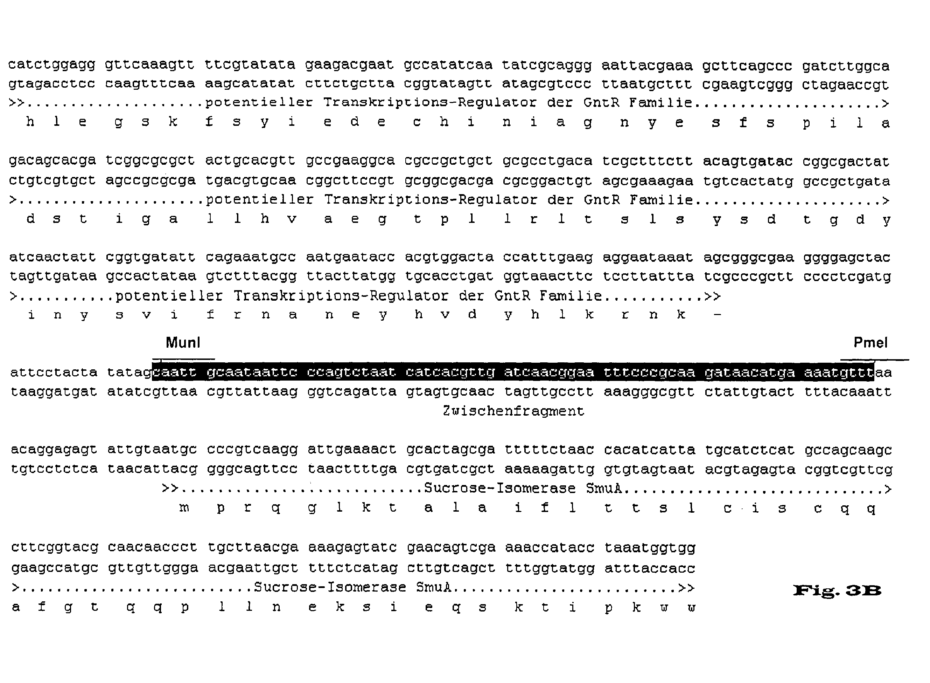

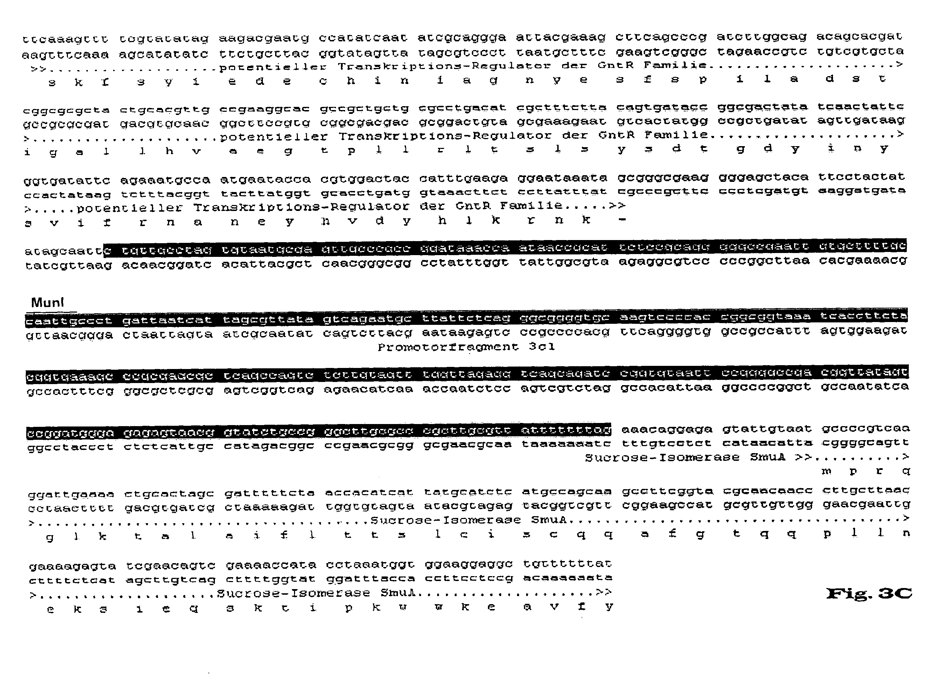

Bevorzugt ist das erfindungsgemäß ausgetauschte oder "ersetzte" *smuA-*Promotorelement in einer *smuA*-Einheit lokalisiert, und zwar bevorzugt in der intergenischen Region zwischen dem Gen *smuA* und einem vorliegend neu gefundenen, stromaufwärts liegenden ORF. Unter einer "*smuA*-Einheit" wird hierin die chromosomale Organisation stromaufwärts des *smuA*-Gens mit regulatorischen Sequenzen in der 5'-UTR (Promotor, Operator) der *smuA*-Expression verstanden. Besonders ist die *smuA*-Einheit charakterisiert durch das Polynucleotid mit der SEQ ID NO: 1 (Figur 1); die Figuren 3ABC illustrieren die bevorzugt auszutauschenden Elemente der intergenischen Region der *smuA*-Einheit von *P. rubrum.*

Die Erfindung sieht bevorzugt eine Wirtszelle vor, die ausgewählt ist aus Mikroorganismen den Gruppen der Gammaproteobacteria und der unklassifizierten Gammaproteobacteria. Vorzugsweise ist die Zelle ausgewählt aus der Gruppe, bestehend aus Mikroorganismen der Gattungen: Escherichia, Salmonella, Serratia, Erwinia, Enterobacter, Klebsiella, Rauoltella, Pectobacterium, Pseudomonas, Azotobacter, Pantoea, Leucanea sowie Protaminobacter. Besonders bevorzugt ist die Zelle ausgewählt aus der Gruppe bestehend aus: *Klebsiella sp.,* besonders Stamm LX3 und Stamm NK33-98-8, *Klebsiella pneumoniae,* besonders Stamm 342; *Enterobacter sp.,* besonders Stamm SZ62; *Enterobacter sp.,* besonders Stamm FMB1; *Rauoltella planticola; Pantoea dispersa; Erwinia rhapontici; Erwinia tasmaniensis,* besonders Stamm Et1/99; *Pectobacterium* atrosepticum, besonders Stamm SCRI 1043; *Pectobacterium carovotum,* besonders Subspezies *brasiliensis,* besonders Stamm PBR 1692; *Protaminobacter rubrum; Pseudomonas mesoacidophila*; *Serratia plymuthica* sowie *Azotobacter vinelandii* und *Leucanea leucocephalia.* In einer besonders bevorzugten Variante ist die Zelle *Protaminobacter rubrum (P. rubrum)* oder ein davon abgeleiteter biotechnologisch einsetzbarer Stamm. Es verteht sich, dass sich die Erfindung auch auf immobilisierte und/oder auf andere Weise modifizierte Formen dieser Wirtszellen, die für biotechnologische Verfahren besonders geeignet sein können, erstreckt.

Gegenstand der Erfindung sind auch Mittel zur Herstellung einer erfindungsgemäßen Zelle, besonders eines Selbstklonierungsstamms. Ein solches Mittel ist ein isoliert vorliegendes vorstehend bereits näher beschriebenes Polynucleotidmolekül als solches, welches den vorstehend charakterisierten Austauschpromotor oder ein funktionelles Fragment davon, das in der Lage ist, die Expression des *smuA*-Gens zu regulieren, und weiter einen die Sucrosemutase kodierenden exprimierbaren Abschnitt, der unter Expressionskontrolle dieses regulierenden Elements steht, enthält oder daraus besteht.

Gegenstand der Erfindung ist auch ein Vektor oder Vektorsystem, besonders ein Austauschplasmid, das geeignet ist, das erfindungsgemäße vorstehend näher charakterisierte Polynucleotidmolekül, das heißt Austauschpromotor oder Austauschpromotorfragment, in eine Zelle einzuschleusen. Die Wirtszelle ist bevorzugt eine der vorstehend charakterisierten Mikroorganismen. Bevorzugt dient der Vektor dazu, den Austauschpromotor beziehungsweise das Austauschpromotorfragment in das chromosomale Genom der Zelle zu integrieren, besonders den nativen Promotor des *smuA*-Gens im Chromosom der Zelle zu ersetzen, um so insbesondere einen Selbstklonierungsstamm zu erzeugen.

Der den Promotoraustausch vermittelnde Austauschvektor ist bevorzugt derart ausgestaltet, dass er in der Wirtszelle, in die er eingebracht wird, nicht repliziert. Ein bevorzugtes Vektorkonstrukt ist ein pUT-Derivat, das auf einem R6K Replikationsursprung basiert; dieser Vektor repliziert, insbesondere nicht in *P. rubrum,* zumindest nicht konditional.

Gegenstand der Erfindung ist auch ein Verfahren zur Herstellung einer erfindungsgemäßen, zur Umwandlung von Saccharose in Isomaltulose oder Isomaltulose-Zusammensetzungen befähigten Zelle, wobei das Verfahren zumindest die folgenden Schritte enthält: In einem ersten Schritt wird ein Wildtypstamm oder ein bereits klassisch oder rekombinant modifizierter Stamm der Zelle bereitgestellt; in einem zweiten Schritt wird dieser Stamm mit mindestens einem der vorstehend charakterisierten erfindungsgemäßen Polynucleotidmoleküle, besonders also dem Austauschpromotor oder Austauschpromotorfragment und/oder mindestens einem der vorstehend charakterisierten Vektoren, enthaltend den Austauschpromotor oder das Austauschpromotorfragment, in Kontakt oder in Verbindung gebracht, und zwar so, bevorzugt dass der Austauschpromotor oder das Austauschplasmid in die Zelle eingeschleust wird. Ein bevorzugtes Verfahren ist die intergenerische Konjugation mit insbesondere *E*. *coli* als Donorstamm.

In einem bevorzugten weiteren, vorzugsweise unmittelbar nachfolgenden Schritt wird die Zelle unter Bedingungen kultiviert, die eine homologe Rekombination zwischen dem eingeschleusten Polynucleotidmolekül beziehungsweise Polynucleotidmolekül-tragenden Vektor oder Austauschplasmid und dem entsprechenden Abschnitt im Genom der Zelle ermöglichen, sodass der Austauschpromotor in das Genom der Zelle integriert wird. In bevorzugter Ausführung wird der Vektor oder Austauschplasmid das den ursprünglich enthaltenen Austauschpromotor nicht mehr aufweist und vorzugsweise den ausgetauschten *smuA*-Promotor enthält, durch Selektion des zweiten Rekombinationsereignisses eliminiert.

Gemäß eines zweiten Aspekts der Erfindung sind Zellen Gegenstand der Erfindung, worin das *smuA-*Gen, besonders unter der Regulation mindestens eines der vorstehend charakterisierten Austauschpromotoren oder deren funktionellen Austauschpromotorfragmenten, alternativ oder bevorzugt zusätzlich, extrachromosomal (episomal) exprimiert wird, sowie Mittel zur Herstellung solcher rekombinanten Zellen. Ein solches Mittel ist ein weiteres Polynucleotidmolekül, welches einen Sucrosemutase kodierenden exprimierbaren Abschnitt in mindestens einer oder in mehrfacher Kopie enthält und zusätzlich zumindest ein weiteres, die Expression dieser Sucrosemutase regulierendes Element in mindestens einer oder in mehrfacher Kopie, insbesondere also ein Promotorelement. Das die Expression des Sucrosemutasegens regulierende Element ist ausgewählt aus der Gruppe der vorstehend charakterisierten erfindungsgemäßen Austauschpromotoren und funktionellen Fragmenten davon. Gegenstand der Erfindung ist somit eine Sucrosemutase-Expressionskassette.

In einer alternativen Variante dieses Aspekts der Erfindung liegt das erfindungsgemäße Polynucleotidmolekül, besonders die Expressionskassette, in isolierter Form vor. Beispielsweise kann es so in eine Wirtszelle in an sich bekannter Weise transferiert werden. Weiter ist vorgesehen, das erfindungsgemäße Polynucleotidmolekül, besonders die Expressionskassette, zur Entwicklung zellfreier Systeme und Biokatalysatoren zu verwenden.

Gegenstand dieses zweiten Aspekts der Erfindung ist auch ein Vektor, besonders ein Expressionsvektor, welcher zumindest eine Kopie des vorstehend charakterisierten Polynucleotidmoleküls, besonders der Expressionskassette, in bevorzugt exprimierbarer Form enthält. Der Expressionsvektor kann vorzugsweise in eine Wirtszelle eingeschleust werden, um das Sucrosemutasegen (*smuA*) bevorzugt episomal zu exprimieren. In einer bevorzugten alternativen Variante dient der erfindungsgemäße Vektor der Einschleusung der Sucrosemutase-Expressionskassette in das chromosomale Genom der Wirtzelle.

Gegenstand des zweiten Aspekts der Erfindung ist auch ein Verfahren zur Herstellung einer erfindungsgemäßen, zur Umwandlung von Saccharose in Isomaltulose oder Isomaltulose-Zusammensetzungen befähigten rekombinanten Zelle, wobei das Verfahren zumindest die folgenden Schritte enthält: In einem ersten Schritt wird ein Wildtypstamm der Zelle bereitgestellt; in einem zweiten Schritt wird dieser Stamm mit mindestens einem der vorstehend charakterisierten erfindungsgemäßen Polynucleotidmoleküle, besonders der Sucrosemutase-Expressionskassette, und/oder mindestens einem der vorstehend charakterisierten Vektoren, besonders des Sucrosemutase-Expressionsvektors in Kontakt gebracht. Vorzugsweise werden dabei das Polynucleotidmolekül und/oder der Vektor in exprimierbarer Form in die Zelle integriert.

In diesem Zusammenhang steht auch die Verwendung der vorstehend charakterisierten erfindungsgemäßen Polynucleotidmoleküle, Austauschpromotoren und deren Fragmente oder vollständige Sucrosemutase-Expressionskassetten, zur Herstellung einer erfindungsgemäßen Zelle; gemäß des ersten Aspekts der Erfindung also ein Selbstklonierungsstamm, gemäß des zweiten Aspekts der Erfindung also eine rekombinante Zelle.

In diesem Zusammenhang steht auch die Verwendung des vorstehend charakterisierten Vektors, gemäß des ersten Aspekts der Erfindung also ein Austauschplasmid, gemäß des zweiten Aspekts der Erfindung also ein Expressionsvektor, zur Herstellung einer solchen Zelle.

Die Erfindung betrifft schließlich auch Verfahren zur biotechnologischen Herstellung von Isomaltulose oder einer Isomaltulose-Zusammensetzung aus dem Substrat Saccharose oder einem Saccharose-haltigen Substrat. Erfindungsgemäß enthält das Verfahren zumindest den Schritt: Kultivieren der erfindungsgemäßen Zelle in einem Kulturmedium, welches das Substrat enthält, und zwar unter Bedingungen, die eine Umsetzung des Substrats in Isomaltulose, bevorzugt unter Einsatz der zelleigenen, homologen Sucrosemutase (SmuA), ermöglichen. Bevorzugt wird in einem bevorzugt nachfolgenden Schritt die Isomaltulose oder die Isomaltulose-Zusammensetzung aus dem Kulturmedium und/oder der Zelle isoliert.

Die Expression der Sucrosemutase in den erfindungsgemäßen Wirtszellen ist vorteilhafterweise von der Saccharosekonzentration unabhängig oder weitgehend unabhängig, und die Erfindung erlaubt die Anzucht der erfindungsgemäßen Wirtszellen, das heißt den Aufbau an Biomasse, in einem Glukose-Medium (C-Quelle).

Im Zusammenhang mit der Erfindung wird unter einer "Isomaltulose-Zusammensetzung" das Isomerisierungsprodukt der Saccharose verstanden. Dieses enthält zum weitaus überwiegenden Anteil Isomaltulose. Weitere Bestandteile sind Trehalulose und Isomelezitose Besonders wird darunter vor allem folgende Zusammensetzung verstanden: 70 bis 90 % Isomaltulose, 5 bis 10 % Trehalulose, 0 bis 0,5 % Isomelezitose, 0 bis 0,2 % Trisaccharide und 0 bis 0,2 % Restsaccharide.

Ein Gegenstand der Erfindung ist demgemäß auch die Verwendung einer erfindungsgemäßen, vorstehend charakterisierten Zelle zur biotechnologischen Herstellung von Isomaltulose oder einer Isomaltulose-Zusammensetzung, bevorzugt aus Saccharose oder einem Saccharose-haltigen Substrat, und bevorzugt nach dem hier beschriebenen Verfahren.

Die Erfindung wird durch die nachfolgenden Figuren und Beispiele näher erläutert, ohne dass diese beschränkend zu verstehen wären:
Figur 1 illustriert die chromosomale Organisation stromaufwärts des *smuA*-Gens mit regulatorischen Sequenzen in der 5'-UTR (Promotor, Operator); SEQ ID NO: 1: In dieser Region konnte ein "Offener Leserahmen" (ORF) von 234 Aminosäuren identifiziert werden. Dieser zeigt signifikante Homologien zu so genannten GntR-Typ Transkriptions-regulatoren; das entsprechende Gen wird nachfolgend als *gntR* bezeichnet. Zwischen *gntR* und *smuA* befindet sich eine ca. 400 bp umfassende intergenische Region, die regulatorische Elemente (Promotor, Operator) der smuA-Expression aufweist (siehe auch Figur 3A).
Figur 2 zeigt beispielhaft Vektorkarten der erfindungsgemäß einsetzbaren Vektoren "high-copy"-Plasmid pUCTT-gusA (Figur 2A) und "low-copy"-Plasmid pSCTT-gusA (Figur 2B) für die Herstellung von *P. rubrum* Promotorgenbanken. Als Promotorreportergen wurde bevorzugt das β-Glucuronidasegen *gusA* verwendet. Bevorzugt wurden die Austauschsequenzen SEQ ID NO: 2 bis SEQ ID NO: 11 (Tabelle 1 A) in Verbindung mit dem Plasmid nach Figur 2A verwendet; die Austauschsequenzen SEQ ID NO: 12 bis SEQ ID NO: 21 (Tabelle 1 B) wurden in Verbindung mit dem Plasmid nach Figur 2B verwendet.
Figur 3 zeigt DNA-Sequenzausschnitte der intergenischen Region zwischen dem neu gefundenen 3'-Bereich des *gntR* Gens, das für einen potentiellen Transkriptionsregulator der GntR-Familie kodiert, und dem 5'-Bereich des Sucrosemutasegens *smuA* aus *P. rubrum.* Diese Figur illustriert auch die erfindungsgemäß bevorzugte Strategie der narbenfreien Insertion von homologen Austauschpromotoren: Eine natürlicherweise ca. 35 bp stromabwärts des putativen *gntR* Gens gelegene *MunI* Schnittstelle wird ausgewählt. Daneben wird vorzugsweise eine unmittelbar vor der natürlichen *smuA* Ribosomenbindungsstelle (RBS) gelegene AAAC Sequenz genutzt, die einen Teil der *PmeI* Schnittstelle GTTT/AAAC darstellt (Figur 3B). Eine episomale Einführung eines *MunI*/*PmeI-*Zwischenfragments (Figur 3B) erlaubt die gerichtete Insertion von Austauschpromotoren. Exemplarisch zeigt Figur 3C die narbenfreie Insertion des *ribB* Promotors hierin auch als 3C1 bezeichnet. Relevante Sequenzen sind in den Fig 3A-C hervorgehoben.
Figur 4 zeigt beispielhaft ein Vektormap des in Zusammenhang mit der Erfindung hergestellten bevorzugten Promotoraustauschplasmids pUT-GntR-Pr3C1-SmuA, enthaltend die Austauschsequenz nach SEQ ID NO: 2.

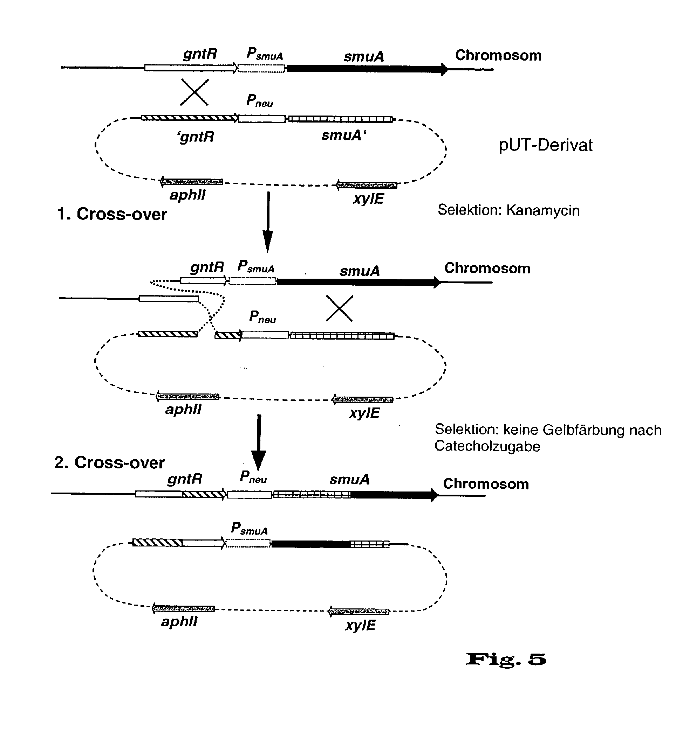

Figur 5 illustriert schematisch und beispielhaft das erfindungsgemäße Vorgehen zur Herstellung der erfindungsgemäßen Organismen: Austausch der nativen *smuA*-Promotorregion durch einen homologen Promotor durch Selbstklonierung. Auf Basis eines in der Wirtszelle *P*. *rubrum* bevorzugt nicht-replikativen Vektors (z.B. pUT-Vektor) wird bevorzugt in *E. coli* ein Promotoraustauschplasmid konstruiert, das den auszutauschenden Promotor enthält. Dieser wird stromaufwärts narbenfrei von einem ca. 1000 bp großen DNA-Fragment, das den putativen *gntR*-Regulator kodiert, und stromabwärts von einem ca. des putativen *gntR*-Regulators kodiert, und stromabwärts von einem ca. 1000 bp großen DNA-Fragment der *SmuA-*Region, das den 5' Bereich von *smuA* kodiert, flankiert. Das erzeugte Plasmid wird in der Wirtszelle, hier: *P. rubrum* Wildtyp, transferiert. Durch Selektion auf vorzugsweise Kanamycin werden daraus solche Kolonien selektioniert, die Austauschplasmide im Chromosom tragen, die vorzugsweise über mindestens eine der beiden homologen Regionen ins Chromosom integrierten. Die Inkubation auf Kanamycin-freiem Medium erlaubt die Selektion von Promotoraustauschstämmen, in denen das integrierte Plasmid über ein zweites Cross-over (zweites Rekombinationsereignis) wieder aus dem Chromosom desintegrierte. Solche Kolonien sind vorzugsweise durch das Fehlen der *xylE*-Aktivität nach Catecholzugabe in an sich bekannter Weise phänotypisch nachweisbar (keine Gelbfärbung). Die erfolgreiche Herstellung des Promotorreorganisationsstammes kann anschließend durch molekularbiologische Methoden (z.B. PCR) in an sich bekannter Weise überprüft werden, um die vollständige Desintegration des Austauschplasmids, die zur Rekonstruktion des Wildtyps führt, zusätzlich ausschließen zu können.

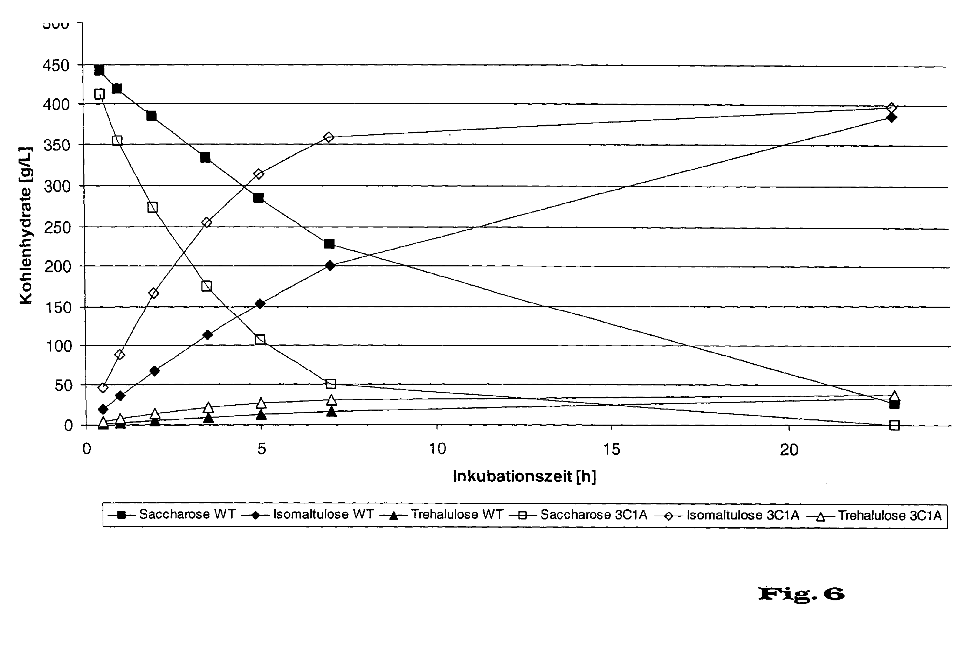

Figur 6 zeigt den Verlauf der Kohlenhydratumsetzung über 24 Studen in erfindungsgemäßen Organismen (Nr. 3C1A) und im Wildtyp (WT) im Vergleich.

Das Sequenzprotokoll enthält:
SEQ ID No:1: DNA-Sequenz stromaufwärts des *smuA*-Gens, die den nativen *smuA*-Promotor/Operator enthält, der durch den erfindungsgemäßen Austauschpromotor oder dessen funktionelles Austauschpromotorfragment vollständig oder teilweise ersetzt werden kann;
SEQ ID No:2 bis 21: Sequenzen funktioneller Austauschpromotoren, die an Stelle des nativen *smuA*-Promotors die Expression des *smuA-*Gens in der Zelle regulieren können.

Um die identifizierten starken Promotoren gegen den nativen *smuA-*Promotor auszutauschen, findet erfindungsgemäß bevorzugt ein Austausch statt, der ohne die Einführung neuer, nicht homologer Sequenzen in dem Organismus durchgeführt wird. Dazu wird erfindungsgemäß bevorzugt im Wesentlichen wie folgt vorgegangen: Ein Promotoraustauschplasmid wird erzeugt, das bevorzugt cirka 1000 bp einer DNA-Region stromaufwärts und stromabwärts des *smuA*-Promotors enthält. Anschließend findet ein narbenfreier Austausch des im Plasmid lokalisierten nativen smuA-Promotors, der eine Größe von cirka 400 bp aufweist, gegen einen anderen homologen Promotor statt. Der basengenaue Austausch kann beispielsweise durch DNA-Sequenzierung bestätigt werden. Das so erhaltene Promotoraustauschplasmid wird in den Organismus transferiert und das Plasmid über die homologe Rekombination in die *smuA*-Einheit chromosomal integriert. Der Promotoraustausch findet bevorzugt statt durch gleichzeitige Eliminierung des Austauschplasmids durch gezielte Selektion des zweiten Rekombinationsereignisses.

Gegebenenfalls kann der korrekte, narbenfreie Promotoraustausch und das Fehlen jedweder Fremdsequenzen im Organismus durch PCR-Verfahren und/oder Sequenzierverfahren in an sich bekannter Weise verifiziert werden. Weitere Absicherungen sind durch Southern-Hybridisierung möglich.

Zum Austausch des nativen *smuA*-Promotors wird bevorzugt die intergenische Region zwischen einem einen potentiellen Regulator der Expression der Saccharosemutase kodierenden Bereich und einem die Saccharosemutase SmuA kodierenden Bereich auf dem Bakterienchromosom ausgewählt. Besonders bevorzugt wird das Zwischenfragment durch Hydrolyse an den Regionen *MunI* und *PmeI* entfernt. Bevorzugt erfolgt die gerichtete Insertion des erfindungsgemäß bevorzugten homologen Austauschpromotors als *MunI* beziehungsweise *EcoRI*/*PmeI*-Fragment.

### Beispiel 1: Austausch des smuA-Promotors durch Selbstklonierung in P. rubrum

Alle Klonierungen und DNA-Modifikationen werden wie unter Sambrook et al., 1989 (Molecular cloning: A laboratory manual, 2nd ed. Cold Spring Harbor Laboratory, Cold Spring Harbor, NY) beschrieben, durchgeführt. PCR-Ansätze, Kits zur Nukleinsäureisolierung, Detektions- und Selektionsverfahren und die Kultivierung wurden in an sich bekannter Weise, soweit nicht anderes angegeben, nach Angaben der jeweiligen Hersteller durchgeführt.

### 1.1 Homologe P. rubrum Promotoren

Chromosomale DNA des *P. rubrum* Wildtyps wird isoliert und partial mit *AluI* verdaut. Die Fragmente werden in die *StuI* Schnittstellen von zwei unterschiedlichen, in Figur 2 dargestellten Promotor-Probe-Vektoren kloniert, die sich im Wesentlichen in der Kopienzahl unterscheiden: Während der Vektor pUCTT-gusA ein pUC-Derivat mit hoher Kopienzahl darstellt und das Chloramphenicol-Resistenzgen *cat* aus pBR328 (DSMZ, Braunschweig) trägt, ist der Vektor pSCTT-gusA ein Vektor mit niedriger Kopienzahl, der vom bekannten Plasmid pSC101 (DSMZ, Braunschweig) abgeleitet wurde und das Kanamycin-Resistenzgen *aphII* trägt. Als Promotor-Reportergen wird bei beiden Vektoren das gusA-Gen eingesetzt, das für einen β-Glucuronidase kodiert und nach Insertion eines funktionalen Promotorfragments exprimiert wird.

Nach Zugabe des Substrats 5-Brom-4-chlor-3-indolyl-β-D-glucuronsäure (X-Gluc) ist eine erfolgreiche Expression durch Blaufärbung detektierbar (Durchführung entsprechend Platteeuw C. et al., 1994, Appl Environ Microbiol. 60:587-93). Durch drei Terminatoren (Ter) kann eine unerwünschte Expression des Reportergens durch potentielle Plasmid-eigene Promotorelemente unterbunden werden. Beide Vektortypen replizieren sowohl in *E*. *coli* als auch *in P. rubrum.*

Zur Überprüfung der Promotorfunktion der wirtszelleigenen Austauschpromotorfragmente kann das in *E. coli* natürlicherweise vorhandene chromosomale β-Glucuronidasegen *uidA* durch dem Fachmann bekannte Methoden inaktiviert werden, um einen *E. coli* gusA-Teststamm *E. coli* DH10BΔ*uid*A zu erzeugen.

Die Transformation der pUCTT-gusA bzw. pSCTT-gusA-Genbank der Austauschpromotoren in *E. coli* DH10BΔ*uidA*, führt zu blauen Kolonien, in denen das promotorlose *gusA*-Reportergen durch das einklonierte Austauschpromotorenfragment exprimiert wird.

Die Restriktionsanalyse von Plasmid-DNA, die von blauen Kolonien isoliert wird, zeigt, dass auch unterschiedlich große chromosomale *P*. *rubrum* Fragmente Promotorenfunktionen aufweisen.

Mit Hilfe der Elektroporation (Präparation elektrokompetenter Zellen und Durchführung nach Dower et al., 1988, Nucleic Acids Res., 16:6127-6145) können die Klone nach *P*. *rubrum* transferiert werden und es kann die bereits in *E*. *coli* detektierte Promotoraktivität bestätigt werden.

### 1.2 Narbenfreie Insertion von Austauschpromotoren

Die erfindungsgemäßen Austauschpromotoren (SEQ ID NO: 2 bis 21) werden jeweils gegen den nativen chromosomalen *smuA-*Promotor/Operator-Bereich (vgl. SEQ ID NO: 1 und Figur 3ABC), narbenfrei durch homologe Rekombination ausgetauscht.

Dieser Ansatz beruht auf chromosomaler Integration eines Promotoraustauschplasmides durch homologe Rekombination. Es wird ein Basisvektor bereitgestellt, der in *P*. *rubrum* zumindest konditional nicht repliziert. Beispielhaft werden pUT-Derivate eingesetzt, das auf einen R6K Replikationsursprung basieren (Herrero et al., 1990, J. Bacteriol., 172:6557-67). Diese Vektoren replizieren nur dann, wenn das für die Replikation essentielle π-Protein kodierende *pir*-Gen anwesend ist. Ein solcher Stamm ist *E. coli* S17-1λpir (Herrero et al., 1990, J. Bacteriol., 172:6557-67), er wird zur Konstruktion der jeweiligen Promotoraustauschplasmide eingesetzt.

Alle auf pUT-Vektor-basierenden erfindungsgemäßen Promotoraustauschplasmide werden so konstruiert, dass das auszutauschende neue Promotorfragment stromaufwärts narbenfrei von einem 925 bp großen DNA-Fragment, das den *gntR*-Regulator kodiert und stromabwärts von einem 1066 bp großen DNA-Fragment, das den 5' Bereich von *smuA* kodiert, flankiert wird. In Figur 3 ist die Realisierung der Klonierungsstrategie beispielhaft für den *ribB*-Promotor Austauschpromotorfragment (3C1) detailliert dargestellt. Ein exemplarisches, finales Promotoraustauschplasmid (pUT-GntR-Pr3C1-SmuA'), das den Austausch des Fragments 3C1 gegen den *smuA-*Promotor vermittelt, ist in Figur 4 dargestellt.

### 1.3 Erzeugen von Promotorreorganisationsstämmen

Der Transfer der hergestellten Promotoraustauschplasmide nach *P*. *rubrum* wird bevorzugt durch eine intergenerische Konjugation zwischen *E. coli* S17-1λpir und *P. rubrum* realisiert. Die eingesetzten pUT-Plasmide tragen einen "Origin of Transfer" (oriT) des RP4 Plasmids und können durch das in *E. coli* S17- *λpir* chromosomal integrierte RP4 Plasmid nach *P. rubrum* mobilisiert werden (Herrero et al., 1990, J. Bacteriol., 172:6557-67).

Die Bedingungen der intergenerischen Konjugation wurden wie folgt optimiert: Die Selektion potentieller *P. rubrum* Transkonjuganten erfordert einen in *P. rubrum* selektionierbaren Plasmidmarker (z.B. *aphII,* Kanamycin-Resistenz), und eine Möglichkeit, den *E. coli* Donor selektiv zu inhibieren. Durch Ausplattieren auf Rifamycin-haltigem Medium (100 µg/ml) werden spontan Rifamycin-resistente *P. rubrum* Wildtypkolonien generiert (*P*. *rubrum* Rif), die sich ansonsten nicht vom Wildtyp unterscheiden. Die Konjugation wird wie folgt ausgeführt:

*E. coli* S17- λ*pir* Donorstämme, die das jeweilige Promotoraustauschplasmid tragen, werden über Nacht in 5 ml dYT Medium (pro 1 Liter: 16 g Bacto Trypton, 10 g Bacto Yeast Extract und 5 g NaCl) unter Zusatz von Kanamycin (50 µg/ml) bei 37°C angezogen. Der Rezipient *P. rubrum* wurde ebenfalls über Nacht in 5 ml dYT unter Zusatz von Rifamycin (100 µg/ml) bei 30°C angezogen. Jeweils 1 ml der Übernachtkultur werden in Erlenmeyerkolben mit 100 ml dYT-Medium (Zusätze siehe oben) überimpft und bei 30°C (P. *rubrum*) bzw. 37°C (*E*. *coli*) und 250 Upm bis zu einer OD (600 nm) von 0,4 bis 0,8 inkubiert. Donor und Rezipient werden im Verhältnis 1:4 gemischt, abzentrifugiert, mit 1 ml dYT gewaschen und final in 100 µl dYT-Medium aufgenommen. Die Suspension wird auf einen Nitrocellulose-Filter (0,45 µm Porengröße) pipettiert, der sich auf einer dYT-Platte befindet und über Nacht bei 30° inkubiert. Die Zellen werden anschließend mit 1 ml dYT vom Filter abgeschwemmt, verdünnt, auf Selektionsplatten ausplattiert (dYT + Kanamycin 50 µg/ml und Rifamycin 100 µg/ml) und über Nacht bei 30°C inkubiert.

Durch die Kanamycin Selektion werden solche *P. rubrum* Transkonjuganten erhalten, in denen die Plasmide vorzugsweise über eine der beiden homologen Regionen ins Chromosom integrierten (Figur 5). Die Inkubation auf Kanamycin-freiem Medium erlaubt die Selektion von Promotoraustauschstämmen, in denen das integrierte Plasmid über ein zweites cross-over aus dem Chromosom desintegriert ist. Zum Nachweis einer erfolgreichen Desintegration kann ein auf den Austauschplasmiden vorhandenes Farbmarkergen *xylE* eingesetzt werden. Das *xylE*-Gen kodiert für eine Catechol-2,3 Dioxygenase, die Catechol zur 2-Hydroxymuconsäure-Semialdehyd umsetzt, was phänotypisch durch markante Gelbfärbung erkennbar ist. Die Desintegration der zuvor integrierten Austauschplasmide ist ein seltenes Ereignis (1 unter 1000 Kolonien) und kann mit Hilfe dieses Markers durch die in den gewünschten Klonen ausbleibende Gelbfärbung nach Catecholzugabe (Sprühreagenz: 0,2 ml einer 0,5 mol/l wässrigen Lösung) detektiert werden. Da die Desintegration des Austauschplasmids auch die Rekonstitution des Wildtyps zur Folge haben könnte, können alle generierten Promotorreorganisationsstämme durch PCR-Experimente, Southern-Blot Analysen und genomische Sequenzierungen überprüft und verifiziert werden. Die Ergebnisse zeigen, dass die generierten neuen Stämme narbenfrei durch basengenauen Austausch (Selbstklonierung) entstanden sind und keinerlei Fremdsequenzen aufweisen.

### Beispiel 2: Produktspektrum und Syntheseleistung

### 2.1 Analyse der Kohlenhydratzusammensetzung mittels HPLC

Die Bestimmung der aufgetrennten Kohlenhydrate erfolgt über HPLC mit folgenden Komponenten: HPLC Pumpe; Probengeber; RI (Brechungsindex)-Detektor; Vorsäule: 10 mm x 4,6 mm, Aminophase (z.B. Zorbax-NH2); Trennsäule: 250 mm x 4,6 mm, Aminophase (z.B. Zorbax-NH2); Interface, Computer und Software zur Messdatenerfassung und -auswertung.

Die Messung wurde unter folgenden chromatographischen Bedingungen durchgeführt: Injektionsvolumen: 10 µl; Flussrate: 1,0 bis 1,8 ml/min. Die einzustellende Flussrate für eine optimale Trennung hängt von Art und Zustand der Trennsäule sowie der Laufmittelzusammensetzung ab. Weitere Analyseparameter siehe Tabelle 2.

**Tabelle 2:**

| | | |
|---|---|---|
| Gerät: | HP1100 HPLC-System | |
| Säule(n): | Zorbax-NH2 250 x 4,6 mm, 5 µm mit Vorsäule 10 x 4,6 mm | bei Raumtemp. |
| Detektor: | Brechungsindex-Detektor | auf 30 °C temperiert |
| Laufmittel: | Acetonitril 73 % (v/v) | Fluß: 1,400 ml/min |
| Injektionsvolumen: | 10 uL | |
| Analysenzeit: | 30 min | |
| Probenkonzentration | 10 %ige Probelösung | |

### 2.2 Ganzzellbiotransformation im Schüttelkolben

Die Anzucht der erfindungsgemäßen Stämme und von Wildtyp-Kontrollstämmen erfolgte in 30 ml LB-Medium (Start-OD₆₀₀ von 0,05). Die Kulturen wurden jeweils bei 30 °C, 200 Upm im Horizontalschüttler inkubiert. Nach zunächst 24 Stunden Fermentation werden 5 x OD Zellen entnommen, abzentrifugiert und mit 1 ml CaAcetat-Puffer (0,01 mol/l, pH 5,5) gewaschen. Die Zellpellets (entsprechend 5 x OD Zellen) wurden jeweils in 1,25 ml CaAcetat-Puffer (0,01 mol/l, pH 5,5) mit einer Saccharoselösung von 0,584 mol/l (200 g/l) resuspendiert. Die Ansätze wurden zu Biotransformation in Deep-Well Platten unter leichtem Schütteln bei Raumtemperatur für 90 min inkubiert. Die Reaktion wurde durch Hitzebehandlung (5 min 98 °C) gestoppt.

**Tabelle 3:**

| Probenbezeichnung | Fructose | Glucose | Saccharose | Isomaltulose | Trehalulose | Isomaltose | DP-3 | Isomelezitose | Rest |
|---|---|---|---|---|---|---|---|---|---|
| | HPLC-NH2 | HPLC-NH2 | HPLC-NH2 | HPLC-NH2 | HPLC-NH2 | HPLC-NH2 | HPLC-NH2 | HPLC-NH2 | HPLC-NH2 |
| | g/L | g/L | g/L | g/L | g/L | g/L | g/L | g/L | g/L |
| 3C1A2S | 4,7 | 4,0 | 63,1 | 138,4 | 10,6 | 0,4 | <0,1 | 0,3 | 0,1 |
| 3C1B2S | 4,6 | 4,0 | 58,2 | 134,1 | 10,2 | 0,4 | <0,1 | 0,3 | <0,1 |
| WT2S | 1,6 | 1,4 | 166,1 | 42,6 | 3,4 | <0,1 | <0,1 | 0,1 | <0,1 |
| 3C1A2G | 4,0 | 3,5 | 78,2 | 111,6 | 8,9 | 0,1 | <0,1 | 0,1 | <0,1 |
| 3C1B2G | 3,8 | 3,3 | 79,0 | 108,4 | 8,4 | 0,2 | <0,1 | 0,1 | <0,1 |
| WT2G | 0,1 | 0,1 | 196,5 | 2,6 | 0,1 | <0,1 | <0,1 | <0,1 | <0,1 |
| | Norm% | Norm% | Norm% | Norm% | Norm% | Norm% | Norm% | Norm% | Norm% |
| 3C1A2S | 2,1 | 1,8 | 28,5 | 62,4 | 4,8 | 0,2 | <0,1 | 0,2 | <0,1 |
| 3C1B2S | 2,2 | 1,9 | 27,5 | 63,3 | 4,8 | 0,2 | <0,1 | 0,1 | <0,1 |
| WT2S | 0,8 | 0,6 | 77,1 | 19,8 | 1,6 | <0,1 | <0,1 | 0,1 | <0,1 |
| 3C1A2G | 1,9 | 1,7 | 37,9 | 54,0 | 4,3 | 0,1 | <0,1 | 0,1 | <0,1 |
| 3C1B2G | 1,9 | 1,6 | 38,9 | 53,3 | 4,2 | 0,1 | <0,1 | <0,1 | <0,1 |
| WT2G | 0,1 | 0,1 | 98,4 | 1,3 | 0,1 | <0,1 | <0,1 | <0,1 | <0,1 |

Tabelle 3 zeigt die Ergebnisse der HPLC-Analyse der Überstände nach Fermentation unter Saccharoseanzucht (S) oder unter Glukoseanzucht (G) bei zwei individuell erzeugten Promotoraustauschstämmen (Nr. 3C1A und Nr. 3C1 B) im Vergleich zum Wildtyp (WT):

Die unter Saccharoseanzucht (S) erzeugte Isomaltulosemenge der 3C1 Stämme (3C1A2S und 3C1B2S) ist gegenüber dem entsprechenden Wildtyp (WT2S) um ca. Faktor 3,2 erhöht.

Unter Glukoseanzucht (G) ist der Unterschied ausgeprägter, da unter diesen Bedingungen SmuA im Wildtyp (WT2G) nur in geringsten Mengen exprimiert wird. Die erfindungsgemäßen Stämme (3C1A2G, 3C1B2G) produzieren im Vergleich zur Saccharoseanzucht nur ca. 10 % weniger Isomaltulose.

Im weiteren Verlauf der Fermentation (länger als 24 Stunden) wird eine Isomaltuloseausbeute von 70 bis 90 % und gegebenenfalls über 90 % erreicht.

### 2.3 Ganzzellbiotransformation im Fermenter

Wildtyp *P*. *rubrum* Z12 und die über Eigenklonierung erzeugten Austauschstämme von *P. rubrum* wurden im gleichen Medium kultiviert. Die Anzucht der Vorkultur erfolgte im Schüttelkolben bei 30 °C unter aeroben Bedingungen.

Vergleichende Fermentationen wurden in 500 ml Fermentern (Firma Sixfors) durchgeführt: Nährmedium: Sojapepton: 15,0 g; Melasse (80 °Bx): 20,0 g; (NH₄)₂HPO₄: 2,0 g; Saccharose: 40,0 g; H₂O ad 1000 ml, pH 7,2-7,4; Fermentationsparameter: Belüftungsrate (Beginn Fermentation): 0,5 VVm; Rührerdrehzahl: 500 Upm; pO₂: 20 %

Die Fermenter wurden mit 5 ml einer in der exponentiellen Wachstumsphase befindlichen Vorkultur beimpft. Die Fermentation in den 500 ml-Fermentern erfolgte unter o.a. Parametern für 15 Stunden bei 30 °C. Nachdem die Fermentationen beendet waren, wurden die Zellen für 30 min bei 17.600x g abzentrifugiert und der klare Überstand verworfen. Anschließend wurde die Zellausbeute (Biotrockenmasse) und deren Sucrosemutaseaktivität bestimmt.

Die Biotrockenmasse wurde durch Filtration von 10 ml Fermentationssupsension über einen 0,45 µm Filter und Trocknung dieses Filters bei 105 °C bestimmt. Es wurden jeweils zwei Fermentationen pro Stamm durchgeführt. Der Wildtyp erzielte nach 15 Stunden Fermentation eine Biotrockenmasse von 85,1 ± 5,6 g/kg und exemplarisch der Promotoraustauschstamm Nr. 3C1 eine Ausbeute von 80,9 ± 1,0 g/kg. Aufgrund dieser Daten konnte kein signifikanter Unterschied in der Biomasseausbeute beobachtet werden.

Zur Bestimmung der Sucrosemutaseaktivität wurden jeweils 1 g Biofeuchtmasse (BFM) des Wildtyps und der erfindungsgemäßen Eigenklonierungsstämme in jeweils 50 g 10 mmol/l Ca-Acetatpuffer, pH 5,5 suspendiert, der 40 % [w/v] Saccharose enthielt. Die Zellsuspension wurde für 24 Stunden bei 25 °C unter Schütteln inkubiert und zu unterschiedlichen Zeitpunkten Proben entnommen. Diese wurden mittels HPLC auf Restsaccharose, Isomaltulose, Trehalulose, Glucose und Fructose untersucht.

In Figur 6 sind Analysendaten zur Bildung von Isomaltulose aus einer 40 %igen Saccharose in 10 mmol/l Ca-acetatpuffer, pH 5,5 dargestellt. Proben wurden zu unterschiedlichen Zeiten entnommen und auf die Zusammensetzung der Kohlenhydrate untersucht. Die Figur zeigt die Umlagerung einer 40 %igen Saccharose zu Isomaltulose durch die Sucrosemutase des Wildtyps (WT) gegenüber dem exemplarisch gewählten Promotoraustauschstamm Nr. 3C1A. Nach 5 Stunden Inkubationszeit konnten mit der Sucrosemutase des Wildtyps 150 g Isomaltulose, mit dem Promotoraustauschstamm Nr. 3C1A aber bereits 320 g Isomaltulose nachgewiesen werden.

Anhand der Umlagerungskinetiken ließen sich folgende spezifische Aktivitäten errechnen: Aktivität des Wildtyps: 1020 ± 17,8 Units/g Biotrockenmasse; Aktivität der Stämme (Nr. 3C1A und Nr. 3C1B): 3118 ± 86,2 Units/g Biotrockenmasse. Die Sucrosemutaseaktivität war in den erfindungsgemäßen Austauschstämmen gegenüber dem Wildtyp um etwa Faktor 3 erhöht.

## Patentansprüche

1. Mikrobielle Zelle mit gegenüber deren Wildtyp mindestens 2-fach gesteigerter Isomaltulose-Syntheserate, wobei die Zelle im Genom ein *smuA*-Gen aufweist, das eine Sucrosemutase SmuA kodiert, wobei mindestens ein *smuA*-Promotorelement durch mindestens einen anderen Promotor (Austauschpromotor) ersetzt ist, der ausgewählt ist aus:
a) einem endogenen Promotor des Gens der Dihydroxy-2 butanon 4-phosphat Synthase (*ribB*)
b) einem zu dem endogenen Promotor nach a) homologen Promotor aus einem fremden Spenderstamm und
c) einem funktionellen Promotorfragment von a) oder b),
wobei der Austauschpromotor durch ein Polynucleotidmolekül charakterisiert ist, das ausgewählt ist aus der Gruppe bestehend aus Polynucleotidmolekülen mit einer Nucleotidsequenz nach SEQ ID NO: 2 bis SEQ ID NO: 3 und homologen Sequenzen davon mit zumindest 85 % Sequenzübereinstimmung.

2. Zelle nach Anspruch 1 mit gegenüber dem Wildtyp mindestens 2-fach gesteigerter Volumenaktivität von Sucrosemutase SmuA.

3. Zelle nach Anspruch 1 oder 2 mit gegenüber dem Wildtyp substratunabhängiger Expression von Sucrosemutase SmuA.

4. Zelle nach einem der Ansprüche 1 bis 3, wobei der ersetzte smuA-Promotor lokalisiert ist in der intergenen Region zwischen smuA und einem stromabwärts liegenden offenen Leseraster (ORF).

5. Zelle nach einem der vorstehenden Ansprüche, wobei die Zelle keine rekombinante genetische Information, heterologe Gene oder Genfragmente enthält oder diese exprimiert.

6. Zelle nach einem der vorstehenden Ansprüche, ausgewählt aus der Gruppe, bestehend aus Mikroorganismen der Gattungen: Escherichia, Salmonella, Serratia, Erwinia, Enterobacter, Klebsiella, Rauoltella, Pectobacterium, Pseudomonas, Azotobacter, Pantoea Leucanea und Protaminobacter.

7. Zelle nach Anspruch 6, ausgewählt aus Organismen der Species *Protaminobacter rubrum.*

8. Isoliertes Polynucleotidmolekül, enthaltend ein die Expression der Sucrosemutase regulierendes Element, das ausgewählt ist aus der Gruppe der in Anspruch 1 charakterisierten Austauschpromotoren, und weiter enthaltend einen die Sucrosemutase kodierenden exprimierbaren Abschnitt, der unter Expressionskontrolle des regulierenden Elements steht.

9. Vektor, enthaltend eines oder mehrere Polynucleotidmoleküle nach der Anspruch 8.

10. Zelle, enthaltend das Polynucleotidmolekül nach Anspruch 8 oder den Vektor nach Anspruch 9.

11. Verfahren zur Herstellung einer in einem der Ansprüche 1 bis 3 charakterisierten Zelle, enthaltend die Schritte:
- Bereitstellen eines Wildtypstamms der Zelle und
- Inkontaktbringen des Stamms mit dem Polynucleotidmolekül nach Anspruch 8 und/oder dem Vektor nach Anspruch 9.

12. Verwendung des Polynucleotidmoleküls nach Anspruch 8 zur Herstellung einer in einem der Ansprüche 1 bis 3 charakterisierten mikrobiellen Zelle.

13. Verfahren zur biotechnologischen Herstellung von Isomaltulose oder einer Isomaltulose-Zusammensetzung aus Saccharose-Substrat, enthaltend den Schritt: Kultivieren der Zelle nach einem der Ansprüche 1 bis 7 oder 10 oder gemäß dem Verfahren nach Anspruch 13 herstellbaren Zelle in einem Kulturmedium, enthaltend das Substrat, unter Bedingungen, die eine Umsetzung des Substrats in Isomaltulose oder eine Isomaltulose-Zusammensetzung ermöglichen.

14. Verwendung der Zelle nach einem der Ansprüche 1 bis 7 oder 10 zur Verbesserung der biotechnologischen Herstellung von Isoma-Itulose oder einer Isomaltulose-Zusammensetzung aus Saccharose-Substrat.

## Claims

1. A microbial cell with an at least 2-fold increased isomaltulose synthesis rate in reference to the wild type, wherein the cell in the genome comprises a smuA-gene coding a sucrose mutase SmuA, with at least one smuA-promoter element being substituted by at least one other promoter (substitute promoter), which is selected from:
a) an endogen promoter of the gene of dihydroxy-2 butanon-4-phosphate synthase (ribB)
b) a promoter of an external donor strain homologue in reference to the endogen promoter according to a) and
c) a functional promoter fragment of a) or b),
wherein the substitute promoter is **characterized by** a polynucleotide molecule selected from a group consisting of polynucleotide molecules with a nucleotide sequence according to SEQ ID NO: 2 to SEQ ID NO: 3 and homologue sequences thereof with at least 85 % sequence identity.

2. Cell according to claim 1 with a volume activity of sucrose mutase SmuA increased at least 2-fold in reference to the wild type.

3. Cell according to claim 1 or 2 with a substrate-independent expression of sucrose mutase SmuA in reference to the wild type.

4. Cell according to one of claims 1 through 3, wherein the substituted smuA-promoter is localized in the inter-genetic region between smuA and a downstream located open reading frame (ORF).

5. Cell according to one of the previous claims, wherein the cell does not comprise any recombinant genetic information, heterologic genes, or gene fragments, or expresses them.

6. Cell according to one of the previous claims, selected from the group consisting of micro-organisms of the genus: escherichia, salmonella, serratia, erwinia, enterobacter, klebsiella, rauoltella, pectobacterium, pseudomonas, azotobacter, pantoea leucanea, and protaminobacter.

7. Cell according to claim 6, selected from organisms of the species protaminobacter rubrum.

8. An isolated polynucleotide molecule, comprising an element regulating the expression of sucrose mutase selected from the group of substitute promoters **characterized in** claim 1, and further comprising a coding section that can express sucrose mutase, its expression being under control of the regulating element.

9. A vector comprising one or more polynucleotide molecules according to claim 8.

10. A cell comprising the polynucleotide molecule according to claim 8 or the vector according to claim 9.

11. A method for the production of a cell **characterized in** one of claims 1 through 3, comprising the steps:
- providing a wild type strain of the cell and
- bringing the strain into contact with the polynucleotide molecule according to claim 8 and/or the vector according to claim 9.

12. Use of the polynucleotide molecule according to claim 8 for the production of a microbial cell **characterized in** one of claims 1 through 3.

13. A method for the biotechnological production of isomaltulose or an isomaltulose-composition from sucrose-substrate, with the step: Cultivating the cell according to one of claims 1 through 7 or 10 or the cell producible by the method according to claim 13 that can be produced in a culture medium, comprising the substrate, under conditions allowing a conversion of the substrate in isomaltulose or an isomaltulose-composition.

14. Use of the cell according to one of claims 1 through 7 or 10 to improve the biotechnological production of isomaltulose or an isomaltulose-composition from sucrose-substrate.

## Revendications

1. Cellule microbienne avec un taux de synthèse d'isomaltulose augmenté au moins deux fois par rapport à son type sauvage, la cellule ayant dans son génome un gène *smuA* qui code une saccharose-mutase SmuA, au moins un élément promoteur de *smuA* étant remplacé par au moins un autre promoteur (promoteur de remplacement) qui est choisi dans :
a) un promoteur endogène du gène de la dihydroxy-2 butanone-4-phosphate synthase (*ribB*),
b) un promoteur d'une souche donatrice étrangère qui est homologue au promoteur endogène selon a), et
c) un fragment de promoteur fonctionnel de a) ou b),
le promoteur de remplacement étant **caractérisé par** une molécule de polynucléotide qui est choisie dans le groupe consistant en des molécules de polynucléotide avec une séquence nucléotidique selon SEQ ID NO. 2 à SEQ ID NO. 3 et leurs séquences homologues avec une identité de séquence d'au moins 85 %.

2. Cellule selon la revendication 1 avec une activité volumique de saccharose-mutase SmuA augmentée au moins deux fois par rapport à son type sauvage.

3. Cellule selon la revendication 1 ou 2, avec, par rapport à son type sauvage, une expression de saccharose-mutase SmuA indépendante de substrat.

4. Cellule selon l'une quelconque des revendications 1 à 3, le promoteur smuA remplacé étant localisé dans la région intergénique entre smuA et un cadre de lecture ouvert (ORF) en aval.

5. Cellule selon l'une quelconque des revendications précédentes, la cellule ne contenant pas ou n'exprimant pas des informations génétiques recombinantes, des gènes ou des fragments de gène hétérologues.

6. Cellule selon l'une quelconque des revendications précédentes, choisie dans le groupe consistant en des microorganismes des genres : Escherichia, Salmonella, Serratia, Erwinia, Enterobacter, Klebsiella, Rauoltella, Pectobacterium, Pseudomonas, Azotobacter, Pantoea Leucanea et Protaminobacter.

7. Cellule selon la revendication 6, choisie dans des organismes de l'espèce *Protaminobacter rubrum.*

8. Molécule de polynucléotide isolée, contenant un élément régulateur de l'expression de la saccharose-mutase, l'élément étant choisi dans le groupe des promoteurs de remplacement caractérisés dans la revendication 1, et en outre contenant une partie exprimable qui code pour la saccharose-mutase et est sous le contrôle d'expression de l'élément régulateur.

9. Vecteur contenant une ou plusieurs molécules de polynucléotide selon la revendication 8.

10. Cellule contenant la molécule de polynucléotide selon la revendication 8 ou le vecteur selon la revendication 9.

11. Procédé pour la production d'une cellule caractérisée selon l'une quelconque des revendications 1 à 3, le procédé contenant les étapes suivantes :
- fournir une souche de type sauvage de la cellule, et
- mettre en contact la souche avec la molécule de polynucléotide selon la revendication 8 et/ou le vecteur selon la revendication 9.

12. Utilisation de la molécule de polynucléotide selon la revendication 8 pour la production d'une cellule microbienne caractérisée selon l'une quelconque des revendications 1 à 3.

13. Procédé pour la production biotechnologique d'isomaltulose, ou d'une composition d'isomaltulose, à partir d'un substrat de saccharose, le procédé contenant l'étape suivante : cultiver la cellule selon l'une quelconque des revendications 1 à 7 ou 10, ou la cellule qui peut être produite selon la revendication 13, dans un milieu de culture contenant le substrat, dans des conditions qui permettent la conversion du substrat en isomaltulose ou une composition d'isomaltulose.

14. Utilisation de la cellule selon l'une quelconque des revendications 1 à 7 ou 10 pour améliorer la production biotechnologique d'isomaltulose, ou d'une composition d'isomaltulose, à partir d'un substrat de saccharose.
